**Europäisches Patentamt**

**European Patent Office**

**Office européen des brevets**

(19)

(11) Veröffentlichungsnummer : **0 475 226 B1**

(12)

# EUROPÄISCHE PATENTSCHRIFT

(45) Veröffentlichungstag der Patentschrift :
**17.08.94 Patentblatt 94/33**

(51) Int. Cl.⁵ : **C07D 277/72**

(21) Anmeldenummer : **91114692.6**

(22) Anmeldetag : **31.08.91**

(54) Verfahren zur Herstellung von 2-Mercapto-benzothiazol.

(30) Priorität : **07.09.90 DE 4028473**

(43) Veröffentlichungstag der Anmeldung :
**18.03.92 Patentblatt 92/12**

(45) Bekanntmachung des Hinweises auf die
Patenterteilung :
**17.08.94 Patentblatt 94/33**

(84) Benannte Vertragsstaaten :
**BE DE FR GB IT**

(56) Entgegenhaltungen :
**EP-A- 0 237 744**
**DE-A- 1 941 379**
**DE-A- 2 652 394**
**US-A- 3 031 073**
**US-A-40 616 466**

(73) Patentinhaber : **Akzo Nobel N.V.**
**Velperweg 76**
**NL-6824 BM Arnhem (NL)**

(72) Erfinder : **Bergfeld, Manfred, Dr.**
**August-Pfefferstrasse 6**
**W-8765 Erlenbach-Mechenhard (DE)**
Erfinder : **Wohlfahrt, Klaus, Dr.**
**Königsberger Strasse 4**
**W-8751 Elsenfeld (DE)**
Erfinder : **Gütlein, Norbert, Dr.**
**Amriswilstrasse 7**
**W-7950 Biberach/Riss (DE)**

(74) Vertreter : **Fett, Günter et al**
**Akzo Patente GmbH,**
**Postfach 10 01 49**
**D-42097 Wuppertal (DE)**

EP 0 475 226 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein Verfahren zur Herstellung von 2-Mercapto-benzothiazol aus Anilin, Schwefelkohlenstoff und Schwefel.

2-Mercapto-benzothiazol (MBT) ist ein technisch sehr wichtiger Ausgangsstoff zur Herstellung von Vulkanisationsbeschleunigern, wie z.B. Dibenzothiazolyldisulfid und Sulfenamiden.

Industriell wird das MBT im allgemeinen nach dem Kelly-Verfahren hergestellt (US-PS 1 631 871), bei dem Anilin, Schwefelkohlenstoff und Schwefel unter erhöhtem Druck bei erhöhten Temperaturen umgesetzt werden. Das dabei resultierende rohe MBT muß anschließend von nicht umgesetzten Ausgangsverbindungen, Zwischenprodukten und Produkten aus Nebenreaktionen gereinigt werden. Die industriell übliche MBT-Reinigung besteht prinzipiell aus einer Umfällung, bei der das Roh-MBT in Natronlauge gelöst wird, und die teerigen Nebenprodukte abdekantiert, abfiltriert oder extrahiert werden. Die wäßrige Natrium-MBT-Lösung wird gegebenenfalls einer weiteren oxidativen Behandlung unterzogen; anschließend wird das MBT mittels Schwefelsäure ausgefällt und abfiltriert (vgl. DE-PS 22 58 484).

Die Nachteile einer derartigen Reinigung bestehen darin, daß die wäßrige Phase ca. 85 kg $Na_2SO_4$ pro 100 kg gebildeten MBT enthält, die mit dem Abwasser entsorgt werden müssen. Pro 100 kg MBT fallen außerdem ca. 15 kg Abfallprodukte (Teere) an, die größtenteils als feste Abfälle, beispielsweise durch Verbrennung, entsorgt werden müssen. Ein beachtlicher Teil der Nebenprodukte gelangt auch ins Abwasser und verursacht dort einen hohen chemischen Sauerstoffbedarf. Der chemische Sauerstoffbedarf (CSB) wird durch Rücktitration von Bichromat in mg/l $O_2$ bestimmt. Die Analysenmethoden sind unterschiedlich und müssen beim Ergebnis mit angegeben werden.

Bei modernen katalytischen Oxidationsverfahren mit Sauerstoff zu MBTS und Sulfenamiden (vgl. DE-PS 31 13 298 bzw. DE-PS 33 25 724) ist darüber hinaus eine hohe Reinheit des MBT erforderlich, weil dann mit sehr geringen Katalysatorkonzentrationen gearbeitet werden kann. Bei den herkömmlichen Herstellungsverfahren für MBT ist eine solche Reinheit nicht gewährleistet.

Die Fachwelt hat sich daher vielfach bemüht, MBT sowohl mit hoher Reinheit als auch mit hoher Ausbeute herzustellen. So beschreibt die US-PS 3,031,073 ein anderes Verfahren zur Herstellung von MBT , wobei man Anilin, Schwefelkohlenstoff und Schwefel unter Druck in zyklischer Fahrweise erhitzt, den Druck reduziert, das MBT aus dem Roh-Produkt entfernt, den Rückstand mit der nötigen Menge an Anilin, Schwefelkohlenstoff und Schwefel mischt und dieses Gemisch zur Bildung von MBT erneut erhitzt. Zur Isolierung von MBT aus dem rohen Reaktionsprodukt soll jede Methode geeignet sein.

Ausführlich widmet sich diese Patentschrift einem Reinigungsverfahren mit Wasser/Schwefelkohlenstoff-Emulsionen, welche im übrigen noch eine oberflächenaktive Substanz enthalten.

Aber gemäß Beispiel 1 der genannten US-Patentschrift 3,031,073 kann auch mit Schwefelkohlenstoff allein als Reinigungsmittel gearbeitet werden. Nach einer Reaktionszeit von 5 Stunden bei 245°C wird die Reaktion durch Entspannen und Entfernen des Reaktionsgemisches aus dem Autoklaven abgebrochen. Dabei wird der durch Schwefelwasserstoff verursachte Druck durch Entspannen auf Atmosphärendruck völlig abgebaut. Zur Reinigung des Rohproduktes wird dieses mit Schwefelkohlenstoff in einem Autoklaven gemischt und das Gemisch auf 140°C erwärmt und dort 30 bis 45 Minuten gehalten. Man erhält schließlich, auf das Reinigungsverfahren bezogen, MBT in einer Ausbeute von 94% und mit einer Reinheit die mit 99,5% angegeben wird. Bei der Nacharbeitung wurde eine Gesamtausbeute von 82% erhalten. Das MBT wies eine Reinheit von 98% auf.

Die DE-OS-26 52 394 betrifft ein Verfahren zur Reinigung von rohem MBT mit Schwefelkohlenstoff. Nach Beispiel 1 läßt man Anilin, Schwefelkohlenstoff und Schwefel 50 Minuten bei 220°C reagieren und entfernt dann den Schwefelwasserstoff. Zur Reinigung wird das flüssige MBT mit kaltem Schwefelkohlenstoff unter Ausbildung von 2 Phasen in Kontakt gebracht, wobei sich ein Schlamm aus MBT und Schwefelkohlenstoff bildet. Das MBT hatte angeblich eine Reinheit von 99,5%, was schon angesichts des genannten Schmelzbereichs von 172 bis 175°C (Literaturwert von reinem MBT: 180 - 182°C) zweifelhaft erscheint. Die Ausbeute betrug etwa 20% und ist damit völlig unwirtschaftlich.

Die Reinheit des MBT wirkt sich bei der Umsetzung zu den genannten Vulkanisationsbeschleunigern aus. Diesbezügliche Versuche ergaben folgendes: Nach US-PS 30 31 073 hergestelltes und gereinigtes, nach drei Verfahrenszyklen erhaltenes MBT führte in Oxidationsreaktionen gemäß DE-PS 31 13 298 bzw. DE-PS 33 25 724 (Herstellung von MBTS bzw. CBS oder TBBS) zu dunklen schmierigen Ablagerungen an Rührerwelle und Reaktorwand. Diese Ablagerungen banden den Katalysator und verlangsamten die Reaktion stark.

Es bestand also ein Bedürfnis nach einem wirtschaftlichen Verfahren zur Herstellung eines sehr reinen MBT in hoher Ausbeute. Die Rückführung der Zwischen- und Nebenprodukte sollte sich nicht negativ auf die Qualität des MBT in der Weise auswirken, daß es in der Oxidationsreaktion zu Dibenzothiazolyl-disulfid bzw. Sulfenamiden keine schmierigen Ablagerungen bildet und die Reaktion stark verlangsamt, was sich in einer geringen Raum/Zeitausbeute auswirkt.

Die Aufgabe der Erfindung bestand darin, ein Verfahren gemäß dem Oberbegriff zur Verfügung zu stellen, das die genannten Probleme löst.

Das erfindungsgemäße Verfahren ist dadurch gekennzeichnet, daß die Reaktion bei einer Verweilzeit im Reaktor von wenigstens 1 Stunde und bei Temperaturen von 220 - 280°C durchgeführt und diese vor Erreichen des Reaktionsgleichgewichtes abgebrochen und beim Abkühlen durch Vermischen des rohen Reaktionsprodukts mit Schwefelkohlenstoff ein möglichst hoher Schwefelwasserstoffdruck bei erhöhter Temperatur aufrechterhalten und der Schwefelwasserstoff erst nach der Kristallisation des 2-Mercaptobenzothiazols vollständig entfernt wird.

Vorzugsweise beträgt die maximale Verweilzeit $t_{max}$ in Abhängigkeit von der Reaktionstemperatur $T_R$ [°K] der Formel

$$t_{max} = 278,3 - 101 \cdot \log T_R \text{ Stunden.}$$

aus der Formel errechnet sich für eine Reaktionstemperatur von 220°C eine maximale Verweilzeit von 6,3 Stunden, für eine Reaktionstemperatur von 250°C eine maximale Verweilzeit von 3,7 Stunden und für eine Reaktionstemperatur von 280°C eine maximale Verweilzeit von 1,3 Stunden.

Verweilzeit wird dabei als die Zeit verstanden, über die das Reaktionsgemisch auf der Reaktionstemperatur gehalten wird. Der Abbruch der Reaktion erfolgt vor Erreichen des Reaktionsgleichgewichts und dementsprechend bei unvollständigem Umsatz und wird bevorzugt durch Vermischen des Reaktionsgemisches mit Schwefelkohlenstoff sehr rasch bewirkt, wobei der im Reaktionsgemisch vorhandene Schwefelwasserstoff noch nicht entfernt wird. Das Reaktionsgleichgewicht liegt dann vor, wenn die Konzentration an MBT konstant bleibt.

Die Aufrechterhaltung eines möglichst hohen Schwefelwasserstoffdruckes bis zur Kristallisation und die in Abhängigkeit von der Reaktionstemperatur begrenzte Verweilzeit führen zu einem Gemisch von Zwischen- und Nebenprodukten, das bei der Rückführung ohne Anpassung der Zusammensetzung des Einsatzgemisches nach einer gewissen Anzahl von Rückführzyclen hinsichtlich der Produkte und ihrer Mengenanteile konstant bleibt. Die maximale Verweilzeit darf auf keinen Fall überschritten werden, weil sich sonst die Zusammensetzung der Zwischen- und Nebenprodukte in der Weise ändert daß sie nicht mehr in MBT umgewandelt werden können, wodurch die Rückführung in den Prozeß beeinträchtigt wird.

Vorzugsweise wird das rohe geschmolzene MBT mit soviel Schwefelkohlenstoff gemischt, daß die Mischung homogen ist.

Durch die Kombination der erfindungsgemäßen Verfahrensmerkmale werden Ausbeuten von ca. 98% bei einer Reinheit von ca. 98% erzielt. Dieses, obwohl nach dem Massenwirkungsgesetz die im Reaktionsgemisch bleibende Menge an Schwefelwasserstoff die Bildung von MBT zurückdrängen müßte.

Bevorzugte Ausführungsformen der einzelnen Merkmale sind bereits in den Unteransprüchen angegeben.

Vorzugsweise setzt man den rückgeführten Zwischen- und Nebenprodukten Anilin, Schwefelkohlenstoff und Schwefel im Molverhältnis von 0,8 - 1,2 : 1 - 2 : 0,8 - 1,2 zu und läßt unter autogenem Druck dieses Reaktionsgemisch reagieren. Optimale Ausbeuten bei sehr hoher Reinheit erhält man, wenn man den rückgeführten Zwischen- und Nebenprodukten Anilin, Schwefelkohlenstoff und Schwefel im Molverhältnis von 0,9 - 1,1 : 1,2 - 1,7 : 0,9 - 1,1 zusetzt und unter autogenem Druck dieses Reaktionsgemisch reagieren läßt. Besonders werden diese optimalen Ergebnisse dann erhalten, wenn man das Reaktionsgemisch bei 245 - 255°C und einer Verweilzeit von 1,5 - 2,5 Stunden unter autogenem Druck umsetzt.

Bei der Mischung des Reaktionsgemisches mit $CS_2$ nach Beendigung der Umsetzung sind die Temperatur und die Konzentration von $CS_2$ so zu wählen, daß eine visuell klare Lösung mit möglichst hoher Konzentration an MBT entsteht. Im allgemeinen kommen auf 1 Gewichtsteil Reaktionsgemisch 0,7 bis 10 Gewichtsteile $CS_2$, bevorzugt 0,9 bis 3. Beispielsweise liegt nach Abbruch der Reaktion die Temperatur des Reaktionsgemisches bei 180 bis 200°C und die des $CS_2$ bei höchstens 100 bis 140°C. Die sich einstellende Temperatur der Lösung soll vorzugsweise oberhalb von 100°C liegen, um eine hohe MBT-Konzentration zu gewährleisten. Zur Herstellung der Lösung kann auch das weitgehend aus $CS_2$ bestehende Waschfiltrat aus der MBT-Reinigungsstufe mit einer Temperatur von beispielsweise 20 - 50°C, im allgemeinen von ca. 35°C ohne weitere Reinigung verwendet werden.

Man kann sowohl das Reaktionsgemisch in einem zweiten Druckgefäß zum $CS_2$ als auch das $CS_2$ zu dem Reaktionsgemisch geben. Bevorzugt ist dabei die Ausführungsform des erfindungsgemäßen Verfahrens, bei der man das rohe Reaktionsgemisch unmittelbar nach Ende der Verweilzeit in ein Druckgefäß überführt, in dem $CS_2$ vorgelegt wurde. Diese Verfahrensweise ermöglicht eine sehr genaue Einhaltung der vorgesehenen Verweilzeit, weil durch die schnelle Abkühlung des Reaktionsgemisches die Reaktion unmittelbar beendet wird und Nachreaktionen nicht mehr ablaufen. Vorzugsweise weist das vorgelegte $CS_2$ eine Temperatur von weniger als 100°C, vorzugsweise zwischen 30 und 100°C auf.

Das Roh-MBT bleibt auch nach dem Mischen mit dem Schwefelkohlenstoff unter dem autogenen Druck des bei der Bildungsreaktion von MBT entwickelten Schwefelwasserstoffs.

Die homogene Mischung wird schließlich vorzugsweise innerhalb eines Zeitraumes zwischen 1,5 und 4,0 Stunden auf eine Temperatur zwischen 20 und 60°C abgekühlt, wobei das reine MBT in feinkörnigen Kristallen anfällt und die nicht umgesetzten Ausgangsverbindungen und die Nebenprodukte sowie geringe Mengen MBT in der Mutterlauge bleiben. $H_2S$ wird erst nach dem Abkühlen der Mischung auf eine Temperatur unterhalb 80°C durch Entspannen vollständig entfernt.

Vorzugsweise wird der im wesentlichen durch Schwefelwasserstoff verursachte Druck durch Entspannen so eingestellt, daß er nach dem Abkühlen auf 150°C noch mindestens 1,5 MPa (15 bar) und nach dem Abkühlen auf 100°C noch mindestens 0,6 MPa (6 bar) beträgt und bei Temperaturen unter 80°C etwa dem Dampfdruck des Schwefelkohlenstoffs entspricht.

Nach dem Abkühlen der Lösung wird das ausgefällte MBT von der Mutterlauge abfiltriert oder abzentrifugiert, mit ca. der 1- bis 3-fachen Menge an Schwefelkohlenstoff gewaschen und unter Stickstoff-Atmosphäre oder evtl. unter Vakuum in einem Trockner bei 50 - 100°C getrocknet.

Die nach der Filtration anfallende Mutterlauge in Form einer Lösung von nicht umgesetztem Anilin und Schwefel sowie den gebildeten Nebenprodukten, aber auch geringen Anteilen MBT, in Schwefelkohlenstoff wird in den Prozeß zur Herstellung von MBT zurückgeführt. Hierzu kann es, je nach Menge des zur Reinigung eingesetzten Schwefelkohlenstoffs, erforderlich sein, die Mutterlauge einzuengen. Man destilliert soviel Schwefelkohlenstoff ab, bis die für den nächsten Ansatz benötigte Menge Schwefelkohlenstoff übrig bleibt. Dieser Schwefelkohlenstoff und die nicht umgesetzten Ausgangsprodukte und Nebenprodukte werden dann in den Reaktor zurückgeführt, dem schließlich noch frisches Anilin und frischer Schwefel zugeführt werden, und zwar in solchen Mengen, daß ohne Berückichtigung der in der Mutterlauge noch vorhandenen Anteile Schwefel und Anilin das für die MBT-Synthese erforderliche Mengenverhältnis der drei Reaktanten wieder eingestellt ist. Das gewonnene $CS_2$ kann zur Herstellung der Lösung oder zum Waschen wiederverwendet werden.

Es wurde nun festgestellt, daß die erfindungsgemäße Verfahrensweise zu einer Beschleunigung des Umsatzes zu MBT führt (s. Beispiel 2). Es kann somit ein autokatalytischer Effekt angenommen werden, weil die erfindungsgemäß erhaltenen Nebenprodukte die vorliegende Bildungsreaktion von MBT offensichtlich beschleunigen. Nicht nur die Anwesenheit von Schwefelwasserstoff, sondern auch die verkürzte Reaktionszeit führt nämlich dazu, daß die Bestandteile der Nebenprodukte des Reaktionsgemisches anders zusammengesetzt sind, als beim Stand der Technik. Dies hat weiterhin den Vorteil, daß die Mutterlauge nicht mit Verunreinigungen aus Neben- oder Zersetzungsreaktionen belastet wird und eine hohe Anzahl an Mutterlauge-Rückführzyklen durchgeführt werden kann. Die Anzahl der Zyklen ist praktisch unbegrenzt, wenn man einen geringen Teil der Mutterlauge mit jedem Zyklus aus dem Kreislauf entfernt. Erfindungsgemäß wird erreicht, MBT mit einer Gesamt-Ausbeute von ca. 98% herzustellen.

Das erfindungsgemäß gereinigte Produkt fällt als feinkörniges, nicht staubendes und leicht rieselndes, also sehr gut handhabbares Material an.

Die vorliegende Erfindung wird durch die nachfolgenden Beispiele näher erläutert:

Beispiel 1

Ein 1,3 Liter Druckautoklav mit Blattrührer wurde jeweils mit folgendem Ansatz beschickt (s. Tab.):

| 153 g | Anilin |
|---|---|
| 52,6 g | Schwefel, süblimiert |
| 187,4 g | Schwefelkohlenstoff (techn.) |
| | Mutterlauge aus vorangegangenem Versuch mit X g festem Rückstand |

Der Reaktor wurde verschlossen und auf 250°C aufgeheizt. Die Verweilzeit betrug 2 Stunden, gemessen ab dem Erreichen der Solltemperatur.

Nach Beendigung der Umsetzung wurde auf 180°C abgekühlt und innerhalb von 20 Minuten wurden 600 g Schwefelkohlenstoff in den Reaktor gepumpt. Nach erfolgter $CS_2$-Zugabe wurde der Reaktor innerhalb von 2,5 Stunden auf 28°C abgekühlt. Erst danach wurde der Druck entspannt und $H_2S$ entfernt. Der Reaktor wurde entleert und die Suspension auf einem Laborfilter getrennt (Vakuumfiltration). Der Filterkurchen wurde schließlich mit ca. 700 g Schwefelkohlenstoff nachgewaschen und anschließend bei 50°C im Vakuum getrocknet.

Das Filtrat wurde im Rotationsverdampfer bei 50°C eingeengt. Sowohl die eingeengte Mutterlauge als auch der zurückgewonnene Schwefelkohlenstoff wurden beim nächsten Ansatz wiederverwendet. Die zu MBT umgesetzten Mengen an Anilin und Schwefel werden ersetzt.

EP 0 475 226 B1

## T a b e l l e 1

| Zyk-lus | Zurück-geführte Nebenpro-dukte | Ausbeute MBT | Reinheit | | Smp | RZA |
|---|---|---|---|---|---|---|
| | | | HPLC | Titr | | |
| | (g) | (g) | Gew.% | Gew.% | (°C) | $kg/m^3h$ |
| 1 | – | 159 g | 99,7 % | 99,4 | 182,7 | 217 |
| 2 | 96,4 | 241,7 | 99,2 | 99,2 | 182,8 | 330 |
| 3 | 125,9 | 281,9 | 98,0 | 98,9 | 182,4 | 383 |
| 4 | 131,2 | 264,0 | 97,7 | 99,0 | 182,3 | 360 |
| 5 | 145,5 | 264,3 | 96,9 | 99,1 | 182,0 | 361 |
| 9 | 188,3 | 271,8 | 97,4 | 98,3 | 181,5 | 371 |
| 13 | 241,1 | 280,5 | 97,0 | 98,9 | 181,1 | 382 |
| 15 | 243,0 | 270,9 | 97,3 | 98,4 | 181,6 | 369 |
| 18 | 239,5 | 265,0 | 97,4 | 98,3 | 180,9 | 362 |

Die Farbe des grobkristallinen MBT reicht von einem sehr hellen Gelb bis zu einem hellen Beige.

**Beispiel 2**

0,2 mol Anilin, 0,2 mol Schwefel und 0,3 mol Schwefelkohlenstoff wurden in einen 100 ml fassenden Druckreaktor gegeben und dort bei 250°C und unter dem sich entwickelnden autogenen Schwefelwasserstoffdruck eine bestimmte, in untenstehender Tabelle angegebene Zeit mit Hilfe eines Magnetrührers gerührt. Nach Ablauf der Verweilzeit wurde der Reaktorinhalt rasch abgekühlt, der Schwefelwasserstoffdruck entspannt und der Reaktorinhalt in Methanol aufgenommen. Die Methanollösung wurde anschließend zur Trockne eingedampft. Der MET-Gehalt des Feststoffs wurde mit Hilfe der HPLC bestimmt und daraus die Gesamtausbeute der Reaktion berechnet.

Unter sonst gleichen Bedingungen wurden weitere Versuche durchgeführt, bei der der Reaktionsmischung jeweils Mutterlauge mit 25 g Zwischen- und Nebenprodukten zugemischt wurde, wie sie bei der in Beispiel 1 beschriebenen Herstellung von 2-Mercaptobenzothiazol anfällt. Der MBT-Gehalt der Mutterlauge wurde bei der Berechnung der Reaktionsausbeute berücksichtigt.

5

T a b e l l e   2

| Reaktions-<br>temperatur | Verweil-<br>zeit<br>(min) | MBT-Ausbeute (%) | |
|---|---|---|---|
| | | mit Mutterlauge-<br>Rückführung | ohne Mutterlauge-Rück-<br>führung (Vergleich) |
| 250°C | 30 | 48 | 19 |
| 250°C | 60 | 77 | 43,5 |
| 250°C | 90 | 87,5 | 64,5 |
| 250°C | 120 | 93,2 | 74 |
| 250°C | 180 | 96.3 | 85,6 |

**Beispiel 3**

| 204,9 g | Anilin |
|---|---|
| 70,5 g | Schwefel |
| 251,3 g | Schwefelkohlenstoff |
| 375 g | Nebenprodukte aus früherem Cyclus |

wurden in einem 1 l Druckautoklaven mit einer Verweilzeit 2 Stunden lang bei 250°C umgesetzt. Als Verweilzeit wird dabei die Zeit nach dem Erreichen der Reaktionstemperatur von 250°C angesehen.

Die heiße Schmelze wurde in einen 2 l fassenden Kristallisator überführt, in dem 760 g Schwefelkohlenstoff mit einer Temperatur 108°C vorgelegt waren. Die Mischtemperatur betrug 145°C, bei der eine meßbare Reaktion nicht mehr stattfindet. Die Überführung der heißen Schmelze erfolgte unter dem vollen autogenen $H_2S$-Druck. Durch Abkühlen mit 0,8 K/min bis auf Zimmertemperatur wurde das MBT auskristallisiert, wobei $H_2S$ allmählich entfernt wurde. Das feste MBT wurde abfiltriert, mit 400 g $CS_2$ nachgewaschen und bei 50°C im Vakuum getrocknet.

**Beispiel 4** (Vergleichsbeispiel)

Die Verfahrensweise von Beispiel 3 wurde wiederholt, wobei jedoch nach einer Verweilzeit von 2 Stunden bei 250°C der unter dem autogenen $H_2S$-Druck von 56 bar stehende Druckautoklav entspannt und damit $H_2S$ vor der Überführung in den Kristallisator entfernt wurde.

**Beispiel 5** (Vergleichsbeispiel)

Die Verfahrensweise von Beispiel 3 wurde wiederholt, wobei jedoch die Verweilzeit bei der Reaktionstemperatur von 250°C 5 Stunden betrug. Die Überführung in den Kristallisator erfolgte auch hier unter dem vollen autogenen $H_2S$-Druck.

Die Produkteigenschaften des in den Beispielen 3, 4 und 5 erhaltenen MBT sind in der Tabelle 3 gegenübergestellt, wobei sich zeigt, daß mit dem erfindungsgemäßen Verfahren MBT nicht nur in hoher Raum-Zeit-Ausbeute sondern auch mit hoher Reinheit anfällt.

**T a b e l l e   3**

|  | Beispiel 3 | Beispiel 4 (Vergleich) | Beispiel 5 (Vergleich) |
|---|---|---|---|
| Auswaage MBT | 361,6 g | 341,1 g | 348,9 g |
| Aussehen | Pulver,gelb | Pulver,braun | Pulver,braun |
| Farbe (Gardner I) (13 % Na-MBT-Lösung | 12 | 18 | 16 |
| Reinheit HPLC | 97,4 Gew.-% | 94,9 Gew.-% | 95,9 Gew.-% |
| Reinheit titrimetr. | 98,8 " | 97,2 " | 97,8 " |
| Schmelzpunkt | 180,6°C | 179,8°C | 179,7·C |
| DSC-Reinheit | 98,9 mol.-% | 97,5 mol.-% | 97,8 mol.-% |

**Patentansprüche**

1. Verfahren zur Herstellung von 2-Mercaptobenzothiazol aus Anilin, Schwefel und Schwefelkohlenstoff unter Druck und Abtrennung des 2-Mercaptobenzothiazol aus dem rohen Reaktionsprodukt durch Abkühlen und Rückführung der Nebenprodukte in den Reaktor zusammen mit Anilin, Schwefel und Schwefelkohlenstoff, dadurch gekennzeichnet, daß die Reaktion bei einer Verweilzeit im Reaktor von wenigstens 1 Stunde bei Temperaturen von 220 - 280°C durchgeführt und diese vor Erreichen des Reaktionsgleichgewichtes abgebrochen und beim Abkühlen durch Vermischen des rohen Reaktionsprodukts mit Schwefelkohlenstoff oberhalb 100°C ein möglichst hoher Schwefelwasserstoffdruck aufrechterhalten und der Schwefelwasserstoff erst nach der Kristallisation des 2-Mercaptobenzothiazols vollständig entfernt wird.

2. Verfahren nach Anspruch 1, dadurch gekennzeichnet, daß die maximale Verweilzeit $t_{max}$ in Abhängigkeit von der Reaktionstemperatur $T_R$ [°K] sich aus der Formel
$$t_{max} = 278,3 - 101 \cdot \log T_R \text{ errechnet.}$$

3. Verfahren nach Anspruch 1 oder 2, dadurch gekennzeichnet, daß man den rückgeführten Zwischen- und Nebenprodukten Anilin, Schwefelkohlenstoff und Schwefel im Molverhältnis von 0,8 - 1,2 : 1 - 2 : 0,8 - 1,2 zusetzt und unter autogenem Druck dieses Reaktionsgemisch reagieren läßt.

4. Verfahren nach Anspruch 3, dadurch gekennzeichnet, daß man den rückgeführten Zwischen- und Nebenprodukten Anilin, Schwefelkohlenstoff und Schwefel im Molverhältnis von 0,9 - 1,1 : 1,2 - 1,7 : 0,9 - 1,1 zusetzt und unter autogenem Druck dieses Reaktionsgemisch reagieren läßt.

5. Verfahren nach einem oder mehreren der Ansprüche 1 bis 4, dadurch gekennzeichnet, daß man das Reaktionsgemisch bei 245 - 255°C und einer Verweilzeit von 1,5 - 2,5 Stunden unter autogenem Druck umsetzt.

6. Verfahren nach einem oder mehreren der Ansprüche 1 bis 5, dadurch gekennzeichnet, daß man das geschmolzene Roh-MBT mit soviel Schwefelkohlenstoff mischt, daß die Mischung homogen ist.

7. Verfahren nach einem oder mehreren der Ansprüche 1 bis 6, dadurch gekennzeichnet, daß man das rohe geschmolzene 2-Mercaptobenzothiazol und den erhitzten Schwefelkohlenstoff derart mischt, daß die Mischung eine Temperatur oberhalb 100 °C aufweist.

8. Verfahren nach einem oder mehreren der Ansprüche 1 bis 7 dadurch gekennzeichnet, daß man das rohe Reaktionsgemisch mit Schwefelkohlenstoff in einem Gewichtsverhältnis von 0,7 bis 10 : 1 und bevorzugt von 0,9 bis 3 : 1 mischt.

9. Verfahren nach einem oder mehreren der Ansprüche 1 bis 8, dadurch gekennzeichnet, daß man das rohe Reaktionsgemisch unmittelbar nach Ende der Verweilzeit in ein Druckgefäß überführt, in dem CS$_2$ vorgelegt wurde.

10. Verfahren nach Anspruch 9, dadurch gekennzeichnet, daß das vorgelegte CS$_2$ eine Temperatur von weniger als 100°C, vorzugsweise zwischen 30 und 100°C aufweist.

11. Verfahren nach einem oder mehreren der Ansprüche 1 bis 10, dadurch gekennzeichnet, daß man das Gemisch aus rohem 2-Mercaptobenzothiazol und Schwefelkohlenstoff innerhalb eines Zeitraums zwischen 1,5 und 4,0 Stunden auf eine Temperatur von 20 - 60°C abkühlen und kristallisieren läßt.

12. Verfahren nach einem oder mehreren der Ansprüche 1 bis 11, dadurch gekennzeichnet, daß der im wesentlichen durch Schwefelwasserstoff verursachte Druck durch Entspannen so eingestellt wird, daß nach dem Abkühlen auf 150°C noch mindestens 1,5 MPa (15 bar) und nach dem Abkühlen auf 100°C noch mindestens 0,6 MPa (6 bar) beträgt und bei Temperaturen unter 80°C etwa dem Dampfdruck des Schwefelkohlenstoffs entspricht.

**Claims**

1. Process for the preparation of 2-mercaptobenzothiazole from aniline, sulphur and carbon disulphide under pressure and the separation of the 2-mercaptobenzothiazole from the crude reaction product by cooling and recycling the by-products into the reactor together with aniline, sulphur and carbon disulphide, characterised in that the reaction is carried out with a residence time in the reactor of at least 1 hour and at temperatures of 220 - 280°C and said reaction is discontinued before the reaction equilibrium is reached and a maximum hydrogen sulphide pressure is maintained on cooling by mixing the crude reaction product with carbon disulphide above 100°C and the hydrogen sulphide is removed completely only after the crystallisation of the 2-mercaptobenzothiazole.

2. Process according to Claim 1, characterised in that the maximum residence time $t_{max}$ is calculated as a function of the reaction temperature $T_R$ [°K] from the formula
$$t_{max} = 278.3 - 101 \cdot \log T_R \text{ hours.}$$

3. Process according to Claim 1 or 2, characterised in that aniline, carbon disulphide and sulphur in a molar ratio of 0.8 - 1.2 : 1 - 2 : 0.8 - 1.2 are added to the recycled intermediates and by-products and this reaction mixture is allowed to react under autogenous pressure.

4. Process according to Claim 3, characterised in that aniline, carbon disulphide and sulphur in a molar ratio of 0.9 - 1.1 : 1.2 - 1.7 : 0.9 - 1.1 are added to the recycled intermediates and by-products and this reaction mixture is allowed to react under autogenous pressure.

5. Process according to one or more of Claims 1 to 4, characterised in that the reaction mixture is reacted at 245 - 255°C and with a residence time of 1.5 - 2.5 hours under autogenous pressure.

6. Process according to one of more of Claims 1 to 5, characterised in that the molten crude MBT is mixed with an amount of carbon disulphide such that the mixture is homogeneous.

7. Process according to one or more of Claims 1 to 6, characterised in that the crude molten 2-mercaptobenzothiazole and the heated carbon disulphide are mixed in such a way that the mixture has a temperature above 100 °C .

8. Process according to one or more of Claims 1 to 7, characterised in that the crude reaction mixture is mixed with carbon disulphide in a weight ratio of 0.7 to 10 : 1 and preferably of 0.9 to 3 : 1.

9. Process according to one or more of Claims 1 to 8, characterised in that the crude reaction mixture is transferred immediately after the end of the residence time into a pressure vessel into which $CS_2$ has been initially introduced.

10. Process according to Claim 9, characterised in that the $CS_2$ initially introduced has a temperature of below 100°C, preferably between 30 and 100°C.

11. Process according to one or more of Claims 1 to 10, characterised in that the mixture of crude 2-mercaptobenzothiazole and carbon disulphide is allowed to cool to a temperature of 20 - 60°C and to crystallise within a period of between 1.5 and 4.0 hours.

12. Process according to one or more of Claims 1 to 11, characterised in that the pressure essentially generated by hydrogen sulphide is so adjusted by letting down that it is still at least 1.5 MPa (15 bar) after cooling to 150°C and still at least 0.6 MPa (6 bar) after cooling to 100°C and at temperatures below 80°C approximately corresponds to the vapour pressure of the carbon disulphide.

**Revendications**

1. Procédé de préparation de 2-mercaptobenzothiazole à partir d'aniline, de soufre et de sulfure de carbone, sous pression, dans lequel le 2-mercaptobenzothiazole est séparé du produit réactionnel brut par refroidissement et les sous-produits sont renvoyés dans le réacteur conjointement avec de l'aniline, du soufre et du sulfure de carbone, ce procédé étant caractérisé en ce que la réaction s'effectue en un temps de séjour dans le réacteur d'au moins 1 heure et à une température de 220°C à 280°C, en ce qu'on interrompt cette réaction avant que soit atteint l'équilibre réactionnel, en ce qu'on maintient une pression de sulfure d'hydrogène la plus élevée possible au-dessus de 100°C, lors du refroidissement qu'on réalise en mélangeant le produit réactionnel brut avec du sulfure de carbone, et en ce qu'on ne chasse totalement le sulfure d'hydrogène qu'après la cristallisation du 2-mercaptobenzothiazole.

2. Procédé conforme à la revendication 1, caractérisé en ce qu'on calcule le temps maximal de séjour $t_{max}$ (en heures) en fonction de la température de réaction $T_R$ (°K), d'après la formule :
$$t_{max} = 278,3 - 101.\log T_R$$

3. Procédé conforme à la revendication 1 ou 2, caractérisé en ce qu'on ajoute, aux produits intermédiaires et aux sous-produits recyclés, de l'aniline, du sulfure de carbone et du soufre, en les rapports molaires 0,8-1,2/1-2/0,8-1,2 et on laisse ce mélange réactionnel réagir sous la pression autogène.

4. Procédé conforme à la revendication 3, caractérisé en ce qu'on ajoute, aux produits intermédiaires et aux sous-produits recyclés, de l'aniline, du sulfure de carbone et du soufre, en les rapports molaires 0,9-1, 1/1,2-1,7/0,9-1,1 et on laisse ce mélange réactionnel réagir sous la pression autogène.

5. Procédé conforme à l'une ou à plusieurs des revendications 1 à 4, caractérisé en ce qu'on fait réagir le mélange réactionnel à une température de 245°C à 255°C, pendant un temps de séjour de 1.5 à 2,5 heures et sous la pression autogène.

6. Procédé conforme à l'une ou à plusieurs des revendications 1 à 5, caractérisé en ce qu'on mélange le 2-mercaptobenzothiazole brut fondu avec une quantité de sulfure de carbone suffisante pour que le mélange soit homogène.

7. Procédé conforme à l'une ou à plusieurs des revendications 1 à 6, caractérisé en ce qu'on mélange le 2-mercaptobenzothiazole brut fondu et le sulfure de carbone chaud de telle façon que la température du mélange soit supérieure à 100°C.

8. Procédé conforme à l'une ou à plusieurs des revendications 1 à 7, caractérisé en ce qu'on mélange le mélange réactionnel brut avec le sulfure de carbone en un rapport pondéral valant de 0,7/1 à 10/1, et de

9

préférence de 0,9/1 à 3/1.

9. Procédé conforme à l'une ou à plusieurs des revendications 1 à 8, caractérisé en ce qu'on transfère le mélange réactionnel brut, immédiatement après la fin du temps de séjour. dans un autoclave où l'on a préalablement placé du sulfure de carbone.

10. Procédé conforme à la revendication 9, caractérisé en ce que la température du sulfure de carbone préalablement placé dans l'autoclave est inférieure à 100°C et se situe de préférence entre 30°C et 100°C.

11. Procédé conforme à l'une ou à plusieurs des revendications 1 à 10, caractérisé en ce qu'on laisse le mélange de 2-mercaptobenzothiazole brut et de sulfure de carbone refroidir jusqu'à une température de 20°C à 60°C, en un laps de temps de 1,5 à 4 heures, tout en laissant la cristallisation s'effectuer.

12. Procédé conforme à l'une ou à plusieurs des revendications 1 à 11, caractérisé en ce qu'on ajuste par détente la pression, exercée essentiellement par du sulfure d'hydrogène, de telle sorte qu'elle vaille encore au moins 1,5 MPa (15 bar) après refroidissement à 150°C et encore au moins 0,6 MPa (6 bar) après refroidissement à 100°C, et qu'aux températures inférieures à 80°C, elle corresponde à peu près à la pression de vapeur du sulfure de carbone.